Europäisches Patentamt

European Patent Office

Office européen des brevets

(11) Numéro de publication: **0 408 109 A1**

## DEMANDE DE BREVET EUROPEEN

(21) Numéro de dépôt: **90201750.8**

(51) Int. Cl.5: **A61F 2/46**

(22) Date de dépôt: **02.07.90**

(30) Priorité: **10.07.89 FR 8909656**

(43) Date de publication de la demande:
**16.01.91 Bulletin 91/03**

(84) Etats contractants désignés:
**AT BE CH DE DK ES FR GB GR IT LI LU NL SE**

(71) Demandeur: **BIOTECNIC SOCIETE ANONYME**
**11 chemin Malepère**
**F-31400 Toulouse(FR)**

(72) Inventeur: **Segrestaa, Daniel**
**156 avenue de Paris**
**F-36000 Chateauroux(FR)**
Inventeur: **Macquart-Moulin, André**
**20 rue Paul Gauguin**
**F-81000 Albi(FR)**
Inventeur: **Carton, François**
**Allée des Tricheries**
**F-31840 Seilh(FR)**

(74) Mandataire: **Barre, Philippe**
**Cabinet Barre-Gatti-Laforgue 95 rue des**
**Amidonniers**
**F-31069 Toulouse Cédex(FR)**

(54) **Dispositif destiné à faciliter l'implantation et à permettre le retrait d'une queue de prothèse de hanche.**

(57) L'invention concerne un dispositif destiné à faciliter l'implantation d'une queue de prothèse de hanche (1) dans une cavité médullaire de fémur, et à permettre le retrait de ladite queue de prothèse. Ce dispositif comprend en premier lieu une prothèse de hanche (1) composée d'une tige fémorale (3) et d'un pivot supérieur (5, 6) et dotée d'une encoche ménagée sur ledit pivot supérieur. Il comprend, en outre, un impacteur-extracteur (2) comportant un corps (9) portant deux mâchoires (15, 19) de formes conjuguées du pivot supérieur (5, 6) de la queue de prothèse (1), adaptées pour emprisonner ledit pivot, lesdites mâchoires comportant un ergot interne (23) en saillie ménagé de façon à se loger dans l'encoche de la queue de prothèse (1), et des moyens (21) de déplacement relatif des deux mâchoires (15, 19).

Fig 1

# DISPOSITIF DESTINE A FACILITER L'IMPLANTATION ET A PERMETTRE LE RETRAIT D'UNE QUEUE DE PROTHESE DE HANCHE

L'invention concerne un dispositif destiné à faciliter l'implantation et à permettre le retrait d'une queue de prothèse de hanche dotée d'un pivot supérieur sur lequel vient s'emmancher une rotule d'articulation. Elle s'étend en tant que moyens essentiels constituant ce dispositif à une queue de prothèse de hanche perfectionnée et à un impacteur-extracteur conçu pour coopérer avec cette queue de prothèse.

Les prothèses de hanche, dites monoblocs, comportant d'un seul tenant une queue de prothèse et une rotule d'articulation, offrent une prise relativement importante qui permet, d'une part, d'ajuster relativement facilement l'orientation de la queue et, d'autre part, le retrait de ces prothèses en cas de nécessité, au moyen d'outils peu complexes tel que celui décrit dans le brevet russe 80-929-088.

Par contre, lors de l'implantation d'une queue de prothèse de hanche destinée à recevoir une rotule d'articulation amovible, l'ajustement précis de l'orientation de la queue selon l'antéversion désirée constitue une source de difficultés, en raison du peu de prise qu'offrent de telles queues de prothèse. Pour la même raison, le retrait de ces queues de prothèse, lorsque cela est nécessaire, pose également des problèmes.

Pour surmonter ces difficultés, plusieurs solutions ont été proposées visant à permettre de saisir la queue de prothèse au moyen d'un instrument adapté. Ainsi, selon le brevet français n° 2.006.925, la prothèse a été dotée d'un oeil ménagé sur l'extrémité d'une languette en saillie sur la face postérieure de la queue. La prothèse peut ainsi être orientée ou retirée au moyen d'un instrument comportant un crochet. Selon le brevet français n° 2.598.609, la queue de prothèse comporte un trou borgne ménagé sur l'appui diaphysaire et formé d'une âme centrale taraudée en vue du vissage d'un outil fileté d'extraction, et d'évidements latéraux en vue de l'introduction d'un outil d'orientation à ergots.

Bien que ces dispositifs permettent de surmonter en partie les difficultés précitées, ils ne permettent toutefois pas de faciliter la prise ferme de la queue de prothèse et donc d'ajuster de façon très précise l'orientation de cette queue dans les meilleures conditions. En outre, lors du retrait, le cas échéant, de la queue de prothèse, l'utilisation de ces instruments peut conduire à soumettre cette queue de prothèse à des efforts de traction ne coïncidant pas avec l'axe diaphysaire de celle-ci et constitue donc une source de risques de traumatismes pour le patient.

Une autre solution, décrite dans le brevet français n° 2.615.097, a consisté à réaliser un impacteur-extracteur assurant le maintien de la prothèse au moyen d'un dispositif de triangulation. Cet impacteur-extracteur comporte à cet effet un pointeau adapté pour venir se loger dans un trou borgne d'impaction, et un collier incliné de 45° par rapport à l'axe du pointeau et adapté pour venir se coincer sur le col de la prothèse, selon le principe d'un serre-joint, sous l'effet de l'effort de traction engendré lors de la pénétration du pointeau dans le trou borgne. Bien que ce type d'impacteur-extracteur assure une prise ferme de la queue de prothèse, il présente toutefois plusieurs inconvénients. En premier lieu, il n'est pas adapté aux prothèses dotées d'un pivot supérieur aminci en vue d'augmenter le débattement cotyloïdien, c'est-à-dire aux prothèses comportant un col cylindrique prolongé d'un pivot conique de diamètre de base supérieur à celui du col. De plus, tout effort exercé sur l'impacteur lors d'une implantation ou d'un retrait de prothèse est transmis par le biais d'un simple filetage, ce qui fragilise cet impacteur. En outre, lors d'une extraction de prothèse, le principe de triangulation conduit à exercer des efforts de flexion importants sur le pointeau, ce qui entraîne fréquemment soit un matage du trou d'impaction et donc une désolidarisation de l'impacteur et de la prothèse, soit une torsion ou même une rupture de l'embout du pointeau. Enfin, malgré la forme spécifique de l'alésage du collier, des risques de détérioration de l'état de surface du col de prothèse subsistent lorsque ce collier est amené à coulisser le long de ce col puis coincé sur ce dernier.

La présente invention vise à pallier ces inconvénients et a pour objectif essentiel de fournir un dispositif composé d'une queue de prothèse et d'un impacteur-extracteur permettant, en combinaison, d'implanter de façon très précise cette queue de prothèse et, le cas échéant, de la retirer aisément sans risque de traumatisme pour le patient.

Un autre objectif est de fournir un dispositif permettant d'excercer des efforts de traction importants sur la queue de prothèse, en vue de son retrait, sans risque de traumatisme pour le patient.

Un autre objectif est de fournir une queue de prothèse dont la forme facilite le positionnement correct et limite les risques de fissuration ou de rupture du ciment.

A cet effet, l'invention concerne un dispositif destiné à faciliter l'implantation d'une queue de prothèse de hanche dans une cavité médullaire de fémur, et à permettre le retrait de ladite queue de prothèse, caractérisé en ce qu'il comprend :

- une prothèse de hanche composée d'une tige fémorale et d'un pivot supérieur et dotée d'une encoche ménagée sur ledit pivot supérieur,
- un impacteur-extracteur comportant :

. un corps portant deux mâchoires de formes conjuguées du pivot supérieur de la queue de prothèse, adaptées pour emprisonner ledit pivot, lesdites mâchoires comportant un ergot interne en saillie de forme conjuguée de l'encoche de la queue de prothèse, disposé de façon à se loger dans ladite encoche lorsque les mâchoires emprisonnent le pivot,

. des moyens de déplacement relatif des deux mâchoires.

L'impacteur-extracteur et la queue de prothèse sont donc conçus pour assurer une prise ferme de cette queue de prothèse permettant d'ajuster sa position de façon très précise et, le cas échéant, d'exercer des efforts de traction importants en vue de son retrait.

D'autre part, l'impacteur-extracteur peut servir de manche universel assurant la prise de prothèses d'essai faisant office de râpes destinées à façonner la cavité médullaire, et introduites, en outre, dans cette cavité en vue de permettre de sélectionner la tête fémorale adéquate.

En outre, selon un mode de réalisation préférentiel :
- le pivot supérieur de la queue de prothèse est agencé de façon que son axe Y forme un angle $\alpha$ par rapport à l'axe diaphysaire de la tige fémorale,
- l'impacteur-extracteur comporte un corps longitudinal et deux mâchoires formant, dans leur position fermée, un angle $\alpha$ avec ledit corps.

Cette disposition permet d'exercer, lors du retrait de la queue de prothèse, des efforts de traction rigoureusement parallèles à l'axe diaphysaire de cette dernière, et supprime donc tout risque de traumatisme.

De plus, le corps de l'impacteur-extracteur est préférentiellement doté d'une tête de préhension présentant une paroi frontale dotée d'un alésage taraudé destiné à loger une extrémité filetée conjuguée d'un accessoire d'extraction de type classique, c'est-à-dire comportant une tige le long de laquelle coulisse une masselotte.

L'impacteur-extracteur peut donc être facilement associé à cet accessoire en vue du retrait de la queue de prothèse, de façon à exercer les efforts de traction nécessaires à ce retrait.

L'invention s'étend également en tant que moyens essentiels constituant ce dispositif, à une queue de prothèse de hanche perfectionnée et à un impacteur-extracteur conçu pour coopérer avec cette dernière.

D'autres caractéristiques, buts et avantages de l'invention ressortiront de la description détaillée qui suit en référence aux dessins annexés qui en représentent, à titre d'exemple non limitatif, un mode de réalisation préférentiel. Sur ces dessins qui font partie intégrante de la présente description :

- la figure 1 est une vue frontale partielle d'un ensemble impacteur-extracteur/queue de prothèse de hanche conforme à l'invention, avec une partie en arraché,
- la figure 2 est une vue en perspective d'une queue de prothèse de hanche conforme à l'invention,
- la figure 3 est une vue externe de cette queue de prothèse, avec une partie en arraché,
- les figures 4a, 4b, 4c et 4d sont des coupes transversales de la tige fémorale respectivement par des plans A, B, C et D,
- la figure 5 est une vue en perspective d'un impacteur-extracteur conforme à l'invention,
- la figure 6 est une coupe longitudinale par un plan axial E de cet impacteur-extracteur.

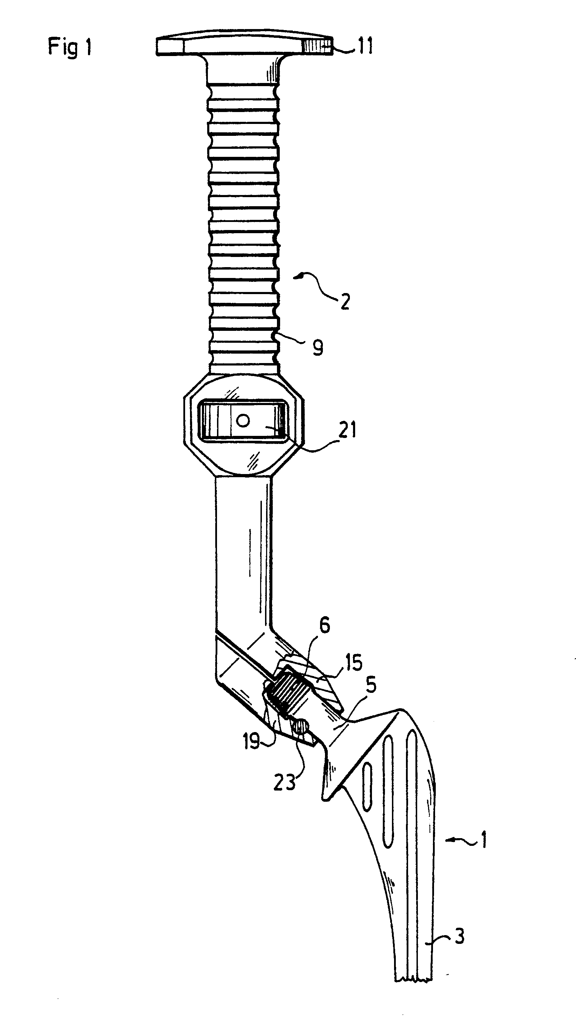

L'ensemble représenté à la figure 1 est formé de deux pièces : une queue de prothèse de hanche 1 destinée à être introduite dans la cavité médullaire d'un fémur et un impacteur-extracteur 2 destiné à coopérer avec cette queue de prothèse lors de son implantation de façon à permettre au praticien d'ajuster de façon précise son orientation selon l'antéversion désirée.

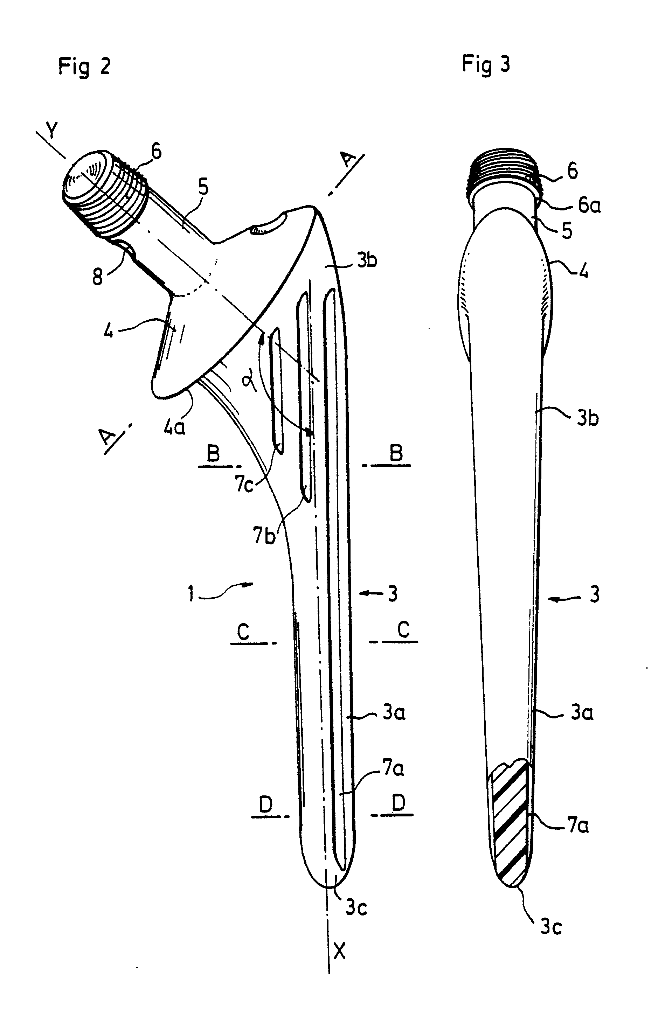

En premier lieu la queue de prothèse 1 représentée aux figures 2, 3, 4, est composée d'une tige fémorale 3, surmontée par une collerette 4 qui se prolonge par un col 5 lui-même prolongé par un pivot conique 6 de diamètre de base supérieur à celui dudit col, délimitant un épaulement 6a avec ce dernier.

L'axe Y du col 5 forme un angle $\alpha$ de 140° avec l'axe diaphysaire X de la tige 3.

En l'exemple la collerette 4 déborde du côté interne pour former un appui 4a avec le calcar. Toutefois selon une variante, cet appui 4a peut être supprimé.

La tige fémorale 3 se compose de deux parties, une partie basse diaphysaire 3a de section circulaire et une partie haute métaphysaire 3b, de section ovoïde. Chaque partie affecte approximativement la moitié de la longueur de la tige fémorale 3.

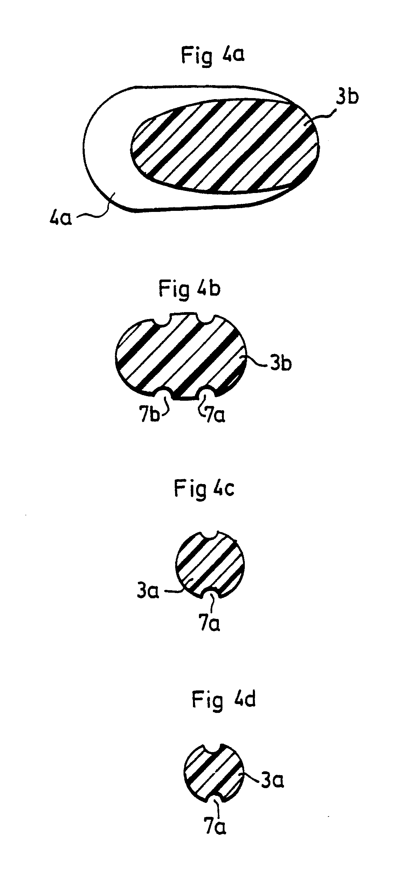

La section ovoïde de la partie haute 3b présente une zone postérieure de largeur supérieure à celle de la zone antérieure. Elle est, en outre, de dimensions croissantes depuis le bas vers le haut, comme le représentent les figures 4a et 4b, et se raccorde sans discontinuité à la section circulaire de la partie basse 3a. Par ailleurs, cette partie basse 3a est également de dimensions croissantes depuis le bas vers le haut.

De plus l'extrémité inférieure 3c de la partie basse 3a est profilée et émoussée de façon à faciliter l'introduction de la tige fémorale 3 et à réduire les douleurs corticales post-opératoires dues aux pressions internes.

En outre, la tige fémorale 3 présente des stries longitudinales 7a, 7b, 7c destinées à faciliter l'échappement du ciment en excès lors de l'implantation. Notamment les stries longitudinales 7a de plus grande longueur sont ainsi ménagées de façon à déboucher au niveau de l'extrémité inférieure 3c de la tige 3 de façon à faciliter l'échappement du ciment en excès dans le fond de la cavité médullaire.

La forme de cette tige fémorale, alliée à une courbure antérieure anatomique, facilite son positionnement correct dans la cavité médullaire lors de l'implantation. En outre, elle réduit considérablement les épaisseurs de ciment mises en place, améliorant les répartitions de contraintes, et réduit fortement les contraintes de cisaillement. Les risques de fissuration ou de ruptures du ciment qui existent avec la majorité des prothèses connues sont ainsi très réduits.

Par ailleurs, le col 5 de cette queue de prothèse 1 présente un diamètre réduit égal à 11,2 mm.

Le pivot conique 6 présente quant à lui une longueur réduite de l'ordre de 10 mm permettant un grand débattement de toute partie cotyloïdienne pouvant être assemblée avec une des têtes fémorales d'une prothèse complète de hanche.

Ce pivot conique 6 présente, en outre, un angle d'ouverture de 10° 20′ ±3′, associé à une diamètre de base, d'entrée de cône, de 13 mm, et un diamètre de sortie de cône de 11,12 mm + 0,03 mm - 0. Ces dimensions permettent de réduire les contraintes radiales d'assemblage et ainsi d'augmenter la résistance mécanique des têtes fémorales assemblées avec cette queue de prothèse. De plus l'angle d'ouverture facilite le désaccouplement, en cas de nécessité de la tête fémorale, réduisant ainsi les risques de descellement de la queue de prothèse lors d'un tel désaccouplement.

Le pivot conique 6 présente enfin un rainurage circulaire périphérique déformable d'une profondeur de 2 à 4/10° de mm. Ce rainurage permet d'amortir l'assemblage et d'impacter en force la tête fémorale de façon à obtenir un maintien par coincement de celle-ci. Il permet, en outre, d'adapter des têtes fémorales de longueurs de cols différents.

En dernier lieu la queue de prothèse 1 comporte une encoche 8 au niveau du col 5, côté antérieur de celui-ci.

Cette encoche 8 permet d'associer à cette queue de prothèse 1, l'impacteur-extracteur 2 décrit ci-dessous en vue d'ajuster de façon précise l'orientation de ladite queue, et de retirer le cas échéant celle-ci.

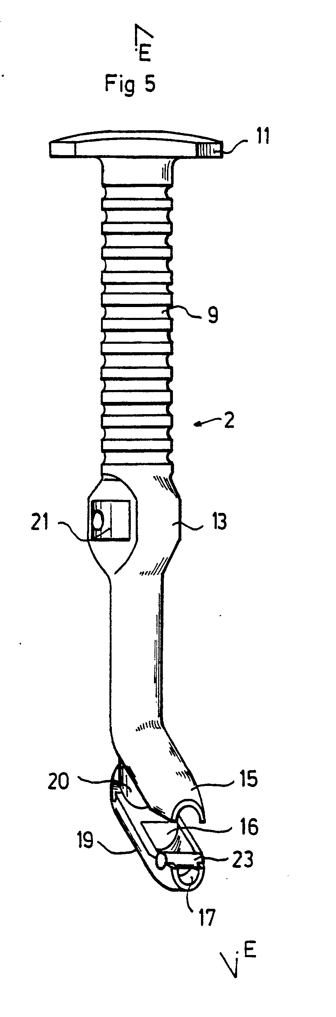

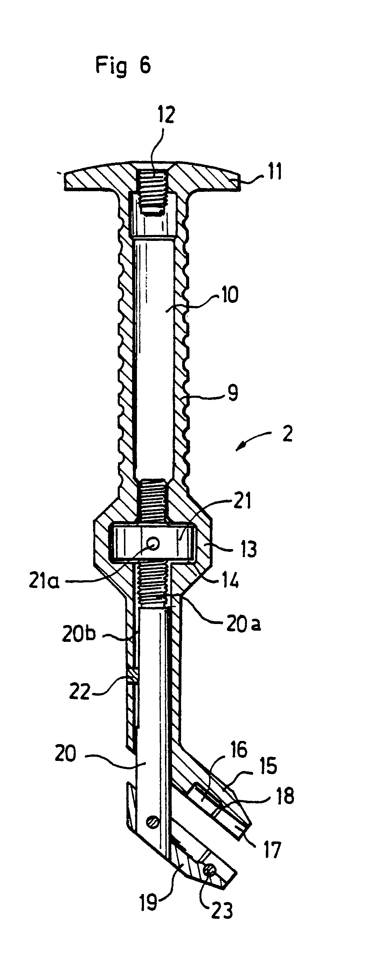

Cet impacteur-extracteur 2 représenté aux figures 5 et 6 comporte un corps tubulaire 9 doté d'un évidement longitudinal cylindrique 10 traversant, obturé vers une des extrémités de ce corps par une tête de préhension 11.

Cette tête de préhension 11 présente une paroi frontale dotée d'un alésage taraudé 12, destiné à recevoir l'extrémité filetée conjuguée d'un accessoire d'extraction de forme classique, c'est-à-dire comportant une tige de long de laquelle coulisse une masselotte, utilisé en vue de l'extraction de la queue de prothèse 1.

Le corps 9 comporte en outre, dans son tronçon médian un renflement 13 délimitant un logement transversal cylindrique débouchant latéralement au niveau de deux faces opposées de ce renflement 13.

Vers l'extrémité opposée à la tête de préhension 11, cet impacteur-extracteur 2 comporte une première mâchoire fixe 15 solidaire du corps 9 et formant un angle de 140° avec l'axe longitudinal de ce dernier.

Cette mâchoire fixe 15 présente une forme interne concave conjuguée de celle du pivot conique 6 et du col 5 de la queue de prothèse 1. Elle présente ainsi deux tronçons semi-cylindriques 16, 17, de diamètres différents, conjugués de ceux du col 5 et de la base du pivot conique 6, séparés par un épaulement 18.

L'impacteur-extracteur 2 comporte une deuxième mâchoire mobile 19 de forme interne symétrique de celle de la mâchoire fixe 15. Cette mâchoire mobile est solidarisée sur l'extrémité d'une tige 20 avec l'axe longitudinal de laquelle elle forme un angle de 140°.

Cette tige 20 présente un diamètre conjugué de celui de l'évidement 10 du corps 9, adapté pour la loger à l'intérieur de cet évidement. Elle est, en outre, associée à des moyens de déplacement axial permettant de la déplacer longitudinalement à l'intérieur de l'évidement 10, et par conséquent d'éloigner ou de rapprocher la mâchoire mobile 19 de la mâchoire fixe 15.

Ces moyens de déplacement axial comprennent un écrou moleté 21 manœuvrable manuellement, disposé dans le logement transversal 14 du corps, et coopérant avec un tronçon fileté 20a de la tige 20 ménagé sur l'extrémité de cette dernière opposée à la mâchoire 19.

Cet écrou moleté 21 est en outre doté radialement de perçages 21a ménagés sur sa paroi périphérique et destinés à loger une tige de serrage permettant d'assurer un serrage adéquat des mâchoires 15, 19.

Afin de bloquer la tige 20 en rotation lors de ses déplacements, cette dernière présente enfin un méplat 20b coopérant avec une clavette 22 solidaire du corps 9.

En dernier lieu la mâchoire 19 comporte, en saillie à l'intérieur du tronçon cylindrique interne 17 de forme conjuguée de celle du col 5, un ergot 23 adapté pour venir coopérer avec l'encoche 8 ménagée sur ce col.

Tel que représenté à la figure 1, cet impacteur-extracteur 2 peut être monté sur la queue de prothèse 1 en vue d'ajuster le positionnement de celle-ci. Dans la position fermée dans mâchoires 15, 19 ces dernières emprisonnent alors le col 5 et le pivot conique 6, et l'ergot 23 logé dans l'encoche 8 assure une prise ferme de la queue de ·prothèse 1.

Le cas échéant, cet impacteur-extracteur 2 peut en outre permettre de retirer la queue de prothèse 1, éventuellement en utilisant un accessoire d'extraction. Lors de ce retrait, les efforts sont exercés parallèlement à l'axe diaphysaire X de la queue de prothèse 1 grâce à l'inclinaison des mâchoires 15, 19 par rapport au corps 9.

Enfin après utilisation la mâchoire 19 et la tige 20 peuvent être facilement démontées en vue du nettoyage et de la stérilisation de l'impacteur-extracteur 2.

**Revendications**

1/ - Dispositif destiné à faciliter l'implantation d'une queue de prothèse de hanche (1) dans une cavité médullaire de fémur, et à permettre le retrait de ladite queue de prothèse, caractérisé en ce qu'il comprend :
- une prothèse de hanche (1) composée d'une tige fémorale (3) et d'un pivot supérieur (5, 6) et dotée d'une encoche (8) ménagée sur ledit pivot supérieur,
- un impacteur-extracteur (2) comportant :
. un corps (9) portant deux mâchoires (15, 19) de formes conjuguées du pivot supérieur (5, 6) de la queue de prothèse (1), adaptées pour emprisonner ledit pivot, lesdites mâchoires comportant un ergot interne (23) de forme conjuguée de l'encoche (8) de la queue de prothèse (1), disposé de façon à se loger dans ladite encoche lorsque les mâchoires emprisonnent le pivot,
. des moyens (20, 21) de déplacement relatif des deux mâchoires (15, 19).
2/ - Dispositif selon la revendication 1 caractérisé en ce que :
- le pivot supérieur (5, 6) de la queue de prothèse (1) est agencé de façon que son axe (Y) forme un angle ($\alpha$) par rapport à l'axe diaphysaire de la tige fémorale (3),
- l'impacteur-extracteur (2) comporte un corps longitudinal (9) et deux mâchoires (15, 19) formant, dans leur position fermée, un angle ($\alpha$) avec ledit corps.
3/ - Dispositif selon l'une des revendications 1 ou 2 caractérisé en ce que :
- le pivot supérieur (5, 6) de la queue de prothèse (1) comporte un col (5) cylindrique sur lequel est ménagée l'encoche (8) et, dans le prolongement dudit col, un pivot conique (6) de diamètre de base supérieur à celui du col (5),
- chaque mâchoire (15, 19) de l'impacteur-extracteur (2) présente une forme interne concave comportant un premier tronçon semi-cylindrique (17) de diamètre conjugué du col (5) et, dans le prolongement de ce premier tronçon, un deuxième tronçon semi-cylindrique (16) de diamètre conjugué du diamètre de base du pivot conique (6).
4/ - Impacteur-extracteur destiné à faciliter l'implantation de la tige fémorale et à permettre le retrait d'une queue de prothèse de hanche (1) comportant une tige fémorale (3) et un pivot supérieur (5, 6), caractérisé en ce qu'il comprend :
- un corps (9) portant deux mâchoires (15, 19) de formes conjuguées du pivot supérieur (5, 6) de la queue de prothèse (1), adaptées pour emprisonner ledit pivot, lesdites mâchoires comportant un ergot interne (23) en saillie,
- des moyens (20, 21) de déplacement relatif des mâchoires (15, 19).
5/ - Impacteur-extracteur selon la revendication 4, caractérisé en ce qu'il comprend :
- un corps (9) longitudinal portant une mâchoire fixe (15) vers une de ses extrémités, ladite mâchoire étant inclinée d'un angle ($\alpha$) par rapport à l'axe longitudinal dudit corps,
- une mâchoire mobile (19) associée à des moyens de déplacement (20, 21) aptes à la déplacer entre une position dite ouverte où elle se trouve à distance de la mâchoire fixe (15) et une position fermée où elle coopère avec ladite mâchoire fixe en formant un angle ($\alpha$) avec le corps (9).
6/ - Impacteur-extracteur selon la revendication 5, caractérisé en ce que :
- le corps (9) présente un évidement longitudinal (10) débouchant au niveau de l'extrémité portant la mâchoire fixe (15),
- les moyens de déplacement comportent une tige (20) logée dans l'évidement (10) et sur une des extrémités de laquelle est solidarisée la mâchoire mobile (19), et des moyens de déplacement axial (20a, 21) de ladite tige à l'intérieur dudit évidement.
7/ - Impacteur-extracteur selon la revendication 6, caractérisé en ce que les moyens de déplacement axial de la tige (20) comprennent un écrou (21) agencé pour coopérer avec un tronçon fileté (20a) ménagé vers l'extrémité de la tige (20) opposée à la mâchoire (19), de façon à entraîner le coulissement longitudinal de cette dernière à l'intérieur de l'évidement (10) du corps (9).
8/ - Impacteur-extracteur selon la revendication 7, caractérisé en ce que la tige (20) comporte un méplat (20b) coopérant avec une clavette (22) solidaire du corps (9).

9/ - Impacteur-extracteur selon l'une des revendications 4 à 8, caractérisé en ce que chaque mâchoire (15, 19) présente une forme interne concave comportant deux tronçons semi-cylindriques (16, 17) de diamètres différents ménagés dans le prolongement l'un de l'autre.

10/ - Impacteur-extracteur selon l'une des revendications 4 à 9, caractérisé en ce que le corps (9) comporte, vers son extrémité opposée à la mâchoire fixe (15), une tête de préhension (11) présentant une paroi frontale dotée d'un alésage taraudé (12) destiné à loger une extrémité filetée conjuguée d'un accessoire d'extraction.

11/ - Queue de prothèse de hanche (1) susceptible d'être implantée ou retirée à l'aide d'un impacteur-extracteur (2) conforme à l'une des revendications 4 à 10, comportant une tige fémorale (3) et un pivot supérieur (5, 6), et caractérisée en ce qu'elle est dotée d'une encoche (8) ménagée sur ledit pivot supérieur.

12/ - Queue de prothèse (1) selon la revendication 11, caractérisée en ce que sa tige fémorale (3) possède une partie basse diaphysaire (3a) de section approximativement circulaire et une partie haute métaphysaire (3b) de section ovoïde se raccordant sans discontinuité à la partie basse circulaire (3a) et s'évasant en direction du pivot supérieur (5, 6), ladite section ovoïde présentant une zone postérieure de largeur supérieure à celle de sa zone antérieure.

13/ - Queue de prothèse selon la revendication 12, caractérisée en ce que :

- la partie basse diaphysaire (3a) présente une section circulaire s'évasant en direction de la partie haute métaphysaire (3b), et comporte une extrémité inférieure (3c) profilée et émoussée,

- la tige fémorale (5) comprend des stries longitudinales (7a, 7b, 7c) d'échappement du ciment lors de l'implantation, deux desdites stries (7a) débouchant au niveau de l'extrémité inférieure (3c).

14/ - Queue de prothèse (1) selon l'une des revendications 11 à 13, caractérisée en ce que le pivot supérieur (5, 6) comporte un col cylindrique (5) sur lequel est ménagée l'encoche (8) et, dans le prolongement dudit col, un pivot conique (6), de diamètre de base supérieur à celui du col (5) de façon à délimiter un épaulement (6a) avec ce dernier.

15/ - Queue de prothèse (1) selon la revendication 14, caractérisée en ce que le pivot conique (6) présente une longueur réduite de l'ordre de 10 mm.

16/ - Queue de prothèse (1) selon l'une des revendications 14 ou 15, caractérisée en ce que le pivot conique (6) présente un angle d'ouverture de $10°20' \pm 3'$ associé à un diamètre de base d'entrée de cône de 13 mm et un diamètre de sortie de cône ce 11,12 mm + 0,3 mm - 0.

17/ - Queue de prothèse (1) selon l'une des revendications 14 à 16, caractérisée en ce que le pivot conique (6) présente un rainurage circulaire périphérique déformable.

Fig 1

Fig 2

Fig 3

Fig 4a

Fig 4b

Fig 4c

Fig 4d

Fig 5

## Fig 6

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | FR-A-2 615 097  (LANDOS APPLICATIONS ORTHOPEDIQUES FRANCAISES) * Page 1, lignes 7-24; page 2, lignes 26-37; page 3, lignes 20-24; page 4, lignes 26-35; page 5, lignes 13-18; figure 1 * | 1-2,4-5 ,11-16 | A 61 F    2/46 |
| A | | 8-9 | |
| D,Y | SOVIET INVENTIONS ILLUSTRATED, semaine 13, 11 mai 1983, section P/Q, classe P31, d'addition E3023K/13, Derwent Publications Ltd, Londres, GB; & SU-A-929 088 (TRAUMATOLOGY ORTHOPAEDIC) 23-05-1982 | 1-2,4-5 ,11-16 | |
| A | DE-B-2 101 002  (AESCULAP-WERKE) * Figure 3 * | 1,4,10 | |
| A | DE-U-8 526 619  (WALDEMAR LINK) * Figure 1 * | 2-3,5-7 | |
| A | FR-A-2 618 667  (J. VEROLA) * Figures 1-2 * | 11-16 | DOMAINES TECHNIQUES RECHERCHES (Int. Cl.5)  A 61 F A 61 B |

Le présent rapport a été établi pour toutes les revendications

| Lieu de la recherche | Date d'achèvement de la recherche | Examinateur |
|---|---|---|
| LA HAYE | 03-10-1990 | NICE P.R. |

EPO FORM 1503 03.82 (P0402)